Europäisches Patentamt

European Patent Office

Office européen des brevets

(11) Numéro de publication : **0 323 287 B1**

(12) **FASCICULE DE BREVET EUROPEEN**

(45) Date de publication du fascicule du brevet :
**15.04.92 Bulletin 92/16**

(51) Int. Cl.$^5$ : **C07C 2/76, B01J 19/24**

(21) Numéro de dépôt : **88403012.3**

(22) Date de dépôt : **30.11.88**

(54) **Procédé de conversion thermique du méthane en hydrocarbures de poids moléculaires plus élevés et réacteur pour la mise en oeuvre du procédé.**

(30) Priorité : **31.12.87 FR 8718524**

(43) Date de publication de la demande :
**05.07.89 Bulletin 89/27**

(45) Mention de la délivrance du brevet :
**15.04.92 Bulletin 92/16**

(84) Etats contractants désignés :
**DE GB IT NL**

(56) Documents cités :
**FR-A- 2 589 859**

(73) Titulaire : **INSTITUT FRANCAIS DU PETROLE**
**4, Avenue de Bois Préau**
**F-92506 Rueil-Malmaison Cédex (FR)**

(72) Inventeur : **Alagy, Jacques**
**24, Chemin Beckensteiner**
**F-69260 Charbonnières (FR)**
Inventeur : **Busson, Christian**
**11, Chemin du Cogny**
**F-69570 Dardilly (FR)**
Inventeur : **Broutin, Paul**
**28, allée Simon Saint Jean**
**F-69130 Ecully (FR)**
Inventeur : **Weill, Jérome**
**16, route des Tourelles**
**F-69005 Lyon (FR)**

EP 0 323 287 B1

Jouve, 18, rue Saint-Denis, 75001 PARIS

## Description

L'invention concerne un procédé amélioré de conversion thermique du méthane en hydrocarbures de poids moléculaires plus élevés et le réacteur pour la mise en oeuvre de ce procédé.

Parmi les sources de méthane se trouvent les gaz naturels et les gaz de raffinerie. Les gaz naturels peuvent être ou non des gaz associés à l'huile brute ; leur composition varie de façon assez sensible suivant leur provenance, mais ils contiennent généralement un pourcentage volumique de méthane compris entre 60 et 95 %. Ce méthane est toujours associé à d'autres alcanes supérieurs, pouvant atteindre et même dépasser des hydrocarbures en $C_6$. Divers procédés cryogéniques permettent de séparer en plusieurs fractions les gaz une fois débarrassés de l'eau et des composants acides : azote, gaz naturels liquéfiés dont on sépare la fraction propane et butane, et une fraction composée essentiellement de méthane associé à une faible quantité d'éthane. Cette dernière fraction est soit réinjectée dans le puits pour maintenir la pression qui fait monter le pétrole brut, soit expédiée par gazoduc comme gaz combustible, soit encore brûlée à la torche.

D'autres sources de méthane sont les gaz de raffinerie qui sont d'origine multiple : gaz de première distillation du pétrole brut, gaz d'hydroréformage, d'hydrotraitements divers, gaz de craquage thermique, gaz de craquage catalytique ; tous ces gaz contiennent dans des proportions diverses, du méthane associé à de nombreux autres composants gazeux, tels que des hydrocarbures légers, de l'azote, du $CO_2$, de l'hydrogène, etc...

C'est ainsi, par exemple qu'un gaz effluent d'une unité de craquage catalytique en lit fluidisé comprend, après lavage, de l'ordre de 30 % en volume de méthane. Ce gaz est souvent fractionné par refroidissement sous pression, permettant d'obtenir deux fractions, l'une comprenant de l'hydrogène, de l'azote, du méthane et une faible partie d'éthylène, l'autre fraction étant composée de la majeure partie de l'éthylène initial, d'éthane, de propane, de propylène.

Cette dernière fraction peut être avantageusement envoyée à une unité de type DIMERSOL, alors que la première est retournée vers le réseau de fuel-gaz de la raffinerie où elle est utilisée comme combustible.

La conversion de méthane en hydrocarbures de poids moléculaires plus élevés présente donc un intérêt certain ; c'est ainsi que, dans les gisements lointains de gaz naturels ou de gaz associés, la conversion du méthane en acétylène, éthylène et aromatiques peut permettre par l'utilisation d'enchainements de procédés connus, d'obtenir des fractions liquides plus facilement transportables et/ou valorisables.

Par exemple, après séparation des solides formés éventuellement, on peut séparer la fraction de composés aromatiques, puis traiter la fraction gazeuse tout d'abord dans des unités permettant l'oligomérisation et/ou la cyclisation de l'acétylène, et ensuite, après une nouvelle séparation gaz/liquides, traiter la fraction gazeuse résiduelle, riche en éthylène, dans des unités de type DIMERSOL, qui permettent d'obtenir des oligomères de l'éthylène.

Sur les lieux mêmes de raffinage, la conversion, même partielle, du méthane en produits plus facilement valorisables présente également un grand intérêt économique.

Il a déjà été proposé différentes méthodes de conversion du méthane ; c'est ainsi que le brevet US 4.199.533 décrit une méthode d'obtention d'éthylène et/ou d'éthane à partir de méthane, consistant à faire réagir sur ce produit du chlore à température supérieure à 700 °C. Ce procédé présente l'inconvénient important de mise en oeuvre à haute température de gaz très corrosifs, comme le chlore et l'acide chlorhydrique.

De nombreux procédés de craquage catalytique du méthane ont été décrits dans l'art antérieur, en utilisant par exemple des catalyseurs de type zéolithique, comme dans le brevet EP 93543, mais tous les catalyseurs utilisés présentent une durée de vie très courte, due aux dépôts de coke formés dans la réaction.

Par ailleurs, le couplage oxydant du méthane est un procédé bien connu, pouvant être conduit soit en présence d'oxygène, soit même en l'absence d'oxygène, des oxydes métalliques intervenant alors dans la réaction en étant réduits ; on peut citer, à titre d'exemples de ces procédés les brevets US 4.172.810, 4.239.658, 4.443.644 et 4.444.984 ; ces produits sont discontinus, puisque l'oxyde métallique doit être régénéré.

Parmi les procédés de craquage thermique susceptibles de transformer du méthane, le procédé dit "procédé WULFF", consiste à utiliser des masses de contact réfractaires ; dans un premier temps on chauffe la masse réfractaire par combustion à l'air d'un combustible qui peut être constitué par la charge elle-même puis, dans un second temps, l'hydrocarbure à craquer est décomposé en absorbant de la chaleur emmagasinée par le matériau réfractaire lors de la période précédente ; il s'agit donc d'un procédé discontinu.

Les procédés à arc électrique et à plasma sont axés essentiellement sur la préparation d'acétylène ; leur consommation élevée en énergie électrique les rend difficilement exploitables.

Il est par ailleurs décrit dans le brevet FR 2.589.859 un système multicanaux réalisé en matière céramique dans lequel les rangées de canaux sont alternativement parcourues par la charge et par les fluides caloporteurs ou réfrigérants, constituant un ensemble continu comportant une zone de pyrolyse suivie d'une zone de trempe. Ce système est toutefois relativement difficile à réaliser.

Un autre type de procédé, quelquefois nommé autothermique, consiste à brûler une partie de la charge

2

pour fournir les calories nécessaires à la réaction de craquage ; ce type de procédé met en oeuvre un brûleur dans lequel environ 1/3 des hydrocarbures est brûlé, le reste étant craqué. Etant donné les hauts niveaux thermiques atteints, ce type de procédé produit essentiellement de l'acétylène et du coke.

Le brevet FR 1.211.695 décrit un procédé de pyrolyse combiné d'hydrocarbures consistant à mélanger du méthane à des gaz de combustion chauds ne comportant pas d'oxygène en excès, puis à injecter dans le mélange obtenu des hydrocarbures paraffiniques à plus d'un atome de carbone ; suivant ce procédé, une très faible partie du méthane peut être transformée en acétylène.

Le couplage thermique deshydrogénant du méthane est fortement endothermique et nécessite l'obtention, à un niveau de température élevé, de l'ordre de 1 100 à 1 500 °C, d'une densité de flux thermique très importante. Il est nécessaire que l'apport maximum de chaleur soit effectué dans la zone où s'effectuent les réactions endothermiques de craquage et de deshydrogénation ; par ailleurs, l'obtention de produits valorisables, tels qu'acétylène, éthylène et/ou composés aromatiques, nécessite un temps de contact très court suivi d'une trempe rapide, de façon à obtenir un profil de température de type "carré".

Il n'existe pas, actuellement de procédé industriel utilisant un transfert de chaleur contrôlé à travers une paroi qui permette de transformer le méthane en hydrocarbure facilement valorisable.

L'objet de cette invention est de pallier cette lacune en proposant un procédé de conversion thermique du méthane en hydrocarbures de poids moléculaires plus élevés, ainsi qu'un réacteur pour la mise en oeuvre de ce procédé conduisant à une mise en oeuvre plus facile, plus souple, et mieux contrôlée.

Plus particulièrement, l'invention concerne un procédé de conversion thermique de méthane en hydrocarbures de poids moléculaires plus élevés dans une zone de réaction allongée ayant un axe de symétrie et comprenant une zone de chauffage et une zone de refroidissement faisant suite à ladite zone de chauffage et dans laquelle on refroidit les effluents de la zone de chauffage, caractérisé en ce que l'on fait circuler un mélange gazeux renfermant du méthane dans ladite zone de chauffage selon une direction d'écoulement sensiblement parallèle à l'axe de ladite zone de réaction, la zone de chauffage contenant une pluralité de moyens de chauffages électriques étanches vis à vis du mélange gazeux disposés en nappes parallèles formant en projection transversale un faisceau à pas triangulaire, carré ou rectangulaire, lesdits moyens de chauffage étant regroupés par sections successives transversales sensiblement perpendiculaires audit axe de symétrie, indépendantes entre elles et alimentées en énergie électrique de façon à déterminer deux parties dans la zone de chauffage, la première partie de la zone permettant de porter la charge jusqu'à une température au plus égale à 1500 °C, la deuxième partie, subséquente de la première, maintenant la charge à une température sensiblement égale à la température maximum à laquelle elle a été portée dans la première partie de façon à ce que la variation de température tout au long de ladite deuxième partie de la zone de chauffage sont inférieure à environ 50°C et de préférence 10°C, le procédé étant aussi caractérisé en ce qu'on introduit un fluide de refroidissement dans ladite zone de refroidissement et en ce qu'on recueille lesdits hydrocarbures de poids moléculaires plus élevés à l'extrémité de la zone réactionnelle.

La zone de chauffage est chauffée par apport d'énergie électrique à travers des moyens de chauffage tels que des résistances électriques ; la chaleur dégagée par effet joule dans ces résistances est transmise principalement par radiation à des gaines en matériau céramique, disposées autour des résistances de façon non jointive. Les charges gazeuses, qui s'écoulent sensiblement perpendiculairement à l'axe des gaines chauffées, sont chauffés essentiellement par convection et par radiation.

Ce type de chauffage statique, par opposition à un chauffage dynamique par circulation d'un fluide caloporteur permet une mise en oeuvre du procédé plus facile et mieux contrôlée.

Selon l'une des caractéristiques principales de l'invention, les résistances électriques qui fournissent de la chaleur à la zone de chauffage sont alimentées de façon indépendante en énergie électrique soit isolément, soit par petits groupes, de façon à définir des sections de chauffage le long de la zone de chauffage et à pouvoir ainsi moduler la quantité d'énergie fournis tout au long de cette zone.

La zone de chauffage est généralement composée de 2 à 20 sections de chauffage, de préférence de 5 à 12. Dans la première partie de cette zone, le mélange gazeux renfermant du méthane, préalablement chauffé à 750 °C environ, est généralement porté rapidement à une température maximale au plus égale à 1 500 °C, et avantageusement entre 1 000 et 1 300 °C. (Le début de la zone de chauffage est située à l'endroit où la charge est introduite).

La modulation de ces sections de chauffage est réalisée de façon classique ; les éléments résistants correspondant aux sections précitées sont en général alimentés par des ensembles modulateurs à thyristors. Des transformateurs permettent éventuellement d'adapter les tensions à priori, alors que les modulateurs permettent le réglage fin et continu de la puissance injectée.

Afin de permettre la régulation de l'ensemble, chaque section de chauffage peut être munie d'une canne pyrométrique à thermocouple adaptée au niveau de température ; ces cannes sont disposées dans les espaces où circule la charge, les informations sont transmises au régulateur qui commande le modulateur à thyristor.

Dans la première partie de la zone de chauffage, l'énergie électrique sert presque exclusivement à porter le mélange réactionnel de sa température initiale -environ 750 °C- à la température de 1 200 °C par exemple, vers laquelle les réactions endothermiques de couplage deshydrogénant du méthane ont lieu. C'est donc au début de la deuxième partie de la zone de chauffage qu'il est nécessaire de fournir l'énergie maximum au milieu réactionnel, ce qui est facilement réalisé par la modulation d'une ou plusieurs sections de chauffage.

La longueur de la première partie de la zone de chauffage représente généralement de 20 à 80 % de la longueur totale de la zone de chauffage, avantageusement de 30 à 70 %.

L'énergie électrique fournie à cette première partie de la zone de chauffage est telle qu'elle génère un fort gradient de température, compris généralement entre 0,5 et 25 °C/cm, et avantageusement entre 1 et 20 °C/cm.

Dans la deuxième partie de la zone de chauffage, on module l'énergie électrique fournie aux différentes sections de chauffage de cette zone de façon à ce que la variation de température tout au long de cette zone soit faible, généralement inférieure à environ 50° C (+ ou - 25°C autour de la valeur de consigne) et avantageusement inférieure à environ 20° C.

La longueur de la zone de chauffage est généralement comprise entre 50 et 90 % de la longueur de la zone réactionnelle totale.

On obtient notamment dans les conditions de chauffage ci-dessus, un flux thermique très important à un niveau de température élevé.

Dans le procédé de l'invention, la zone de chauffage est suivie d'une zone de refroidissement (ou trempe) de façon à abaisser trés rapidement la température des effluents de la zone de chauffage vers environ 300 °C par exemple .

Selon un mode de réalisation, on procède à une trempe directe ; les effluents de réaction quittent la zone de chauffage et sont très rapidement refroidis par une mise en contact direct avec un fluide de refroidissement qui est injecté dans les effluents au moyen d'au moins un injecteur généralement en matériau céramique disposé à la périphérie du réacteur. On peut utiliser, comme fluide de refroidissement des gaz de pétrole liquéfiés, du propane, des huiles hydrocarbonées ou de l'eau. Le propane est le gaz de trempe préféré car il peut également être partiellement craqué et contribuer ainsi à la formation de produits tels que d'éthylène. Les effluents totaux résultant du mélange sont ensuite recueillis et séparés.

Mais ces effluents peuvent aussi être refroidis par contact indirect selon un autre mode de réalisation préféré, le fluide de refroidissement circulant par exemple dans des conduits étanches à l'intérieur de la zone de refroidissement.

L'utilisation de matériaux céramiques comme moyens de chauffage permet d'atteindre des températures de parois de l'ordre de 1 500 °C en utilisation continue, ce qui dépasse les limites de la métallurgie actuelle et permet d'augmenter à la fois la température de mise en oeuvre de la réaction ainsi que la densité de flux thermique.

Par ailleurs, l'utilisation de différentes sections transversales de chauffage, indépendantes les unes des autres, au niveau de la deuxième partie de la zone de chauffage, permet d'apporter le maximum d'énergie thermique à l'endroit où s'effectue la plus grande partie des réactions de craquage endothermiques, et de maintenir dans le reste de la zone de chauffage une température quasi uniforme.

L'ensemble de ces caractéristiques permet d'obtenir, grâce à ce procédé, une conversion thermique du méthane en acétylène, éthylène et produits benzéniques s'effectuant avec un bon taux de conversion et une sélectivité élevée.

Les charges hydrocarbonées utilisables dans le cadre de l'invention sont des charges gazeuses dans les conditions normales de température et de pression, comprenant un pourcentage molaire de méthane compris entre 10 et 99 %, par exemple entre 20 et 99 % et préférentiellement entre 30 et 80 %.

Le reste de la charge peut être constitué par des hydrocarbures aliphatiques, saturés ou non, comprenant un nombre d'atomes de carbone égal ou supérieur à deux, tels que par exemple, l'éthylène, l'éthane, le propane ou le propylène ; d'autres éléments gazeux constitutifs de la charge peuvent être l'azote, le gaz carbonique ou l'oxyde de carbone ou, de préférence l'hydrogène, dont la présence permet de réduire la formation de coke. La proportion molaire d'hydrogène peut être de 1 à 90 %.

On peut, en restant dans le cadre de l'invention, rajouter aux charges définies ci-dessus de la vapeur d'eau de dilution ; le rapport pondéral de la vapeur d'eau de dilution à la charge hydrocarbonée est de l'ordre de 0,1 à 1.

Les charges à traiter ont un temps de séjour dans la zone réactionnelle généralement compris entre 2 et 1 000 millisecondes et de préférence entre 30 et 300 millisecondes.

L'invention concerne aussi le dispositif pour la mise en oeuvre du procédé. Ce dispositif peut être également utilisable pour tout procédé de vapocraquage d'un hydrocarbure contenant au moins deux atomes de carbone. Il est également utilisable pour la réalisation de réactions endothermiques s'effectuant habituellement à des températures supérieures à environ 600 °C, et par exemple 700 à 1 450 °C, avec des temps de séjour de l'ordre de 2 à 1 000 ms.

Plus particulièrement, l'invention concerne un dispositif comprenant des moyens d'alimentation en mélange gazeux et des moyens d'évacuation des effluents produits, caractérisé en ce qu'il comprend un réacteur 1 de forme allongée, de préférence à section carrée ou rectangulaire, en matière réfractaire, ayant un axe de symétrie et relié d'une part, à une première extrémité, auxdits moyens d'alimentation et d'autre part, à l'extrémité opposée, auxdits moyens d'évacuation, ledit réacteur comprenant dans une première zone de chauffage une pluralité de moyens de chauffage électrique 3 entourés de façon non jointive, mais étanches aux gaz, de gaines en céramique 4, lesdits moyens sensiblement parallèles entre eux, étant disposés en nappes sensiblement parallèles, perpendiculaires à l'axe de symétrie du réacteur, de façon à définir entre les gaines des espaces pour la circulation des mélanges gazeux et/ou des effluents, ledit réacteur comportant en outre des moyens d'asservissement et de modulation de chauffage permettant de chauffer lesdites gaines par sections transversales successives indépendantes sensiblement perpendiculaires à l'axe du réacteur, ledit réacteur comportant par ailleurs dans une deuxième zone, continue à la première, des moyens de refroidissement des effluents, adaptés à refroidir par contact direct ou par contact indirect les effluents quittant la première zone.

Les gaines peuvent être disposées de façon superposée ou en quinconce et peuvent former en projection transversale un faisceau à pas triangulaire ou à pas carré.

Le nombre total de nappes des moyens de chauffage, et le nombre des moyens de chauffage par nappe ne sont pas déterminants dans le procédé ; ils sont évidemment fonction de la dimension de l'ensemble du réacteur ainsi que des dimensions des moyens de chauffage et des gaines en céramique qui les entourent. Les éléments de chauffage peuvent être identiques entre eux ou différents, tant par leurs dimensions que par leur puissance de chauffage.

Le nombre d'éléments chauffants détermine la puissance électrique maximum disponible pour un volume réactionnel donné, et influe également sur le temps de séjour de la charge ; il sera choisi en fonction du débit de charge admissible, compte tenu de ces paramètres.

On peut, dans le cadre de l'invention, réaliser l'ensemble du réacteur zone de chauffage et zone de trempe sous forme d'un monobloc, soit encore par juxtaposition jointive de divers éléments de forme identique, qui sont assemblés entre eux par tout moyen utilisable comme, par exemple, à l'aide de brides.

Les moyens de chauffage électriques utilisables dans le cadre de l'invention sont de préférence des résistances chauffantes susceptibles d'être utilisées jusqu'à des températures de l'ordre de 1 500 °C ; on préfère utiliser des résistances en carbure de silicium, de forme cylindrique.

Les gaines en céramique qui entourent ces résistances, de façon à éviter un contact direct entre les mélanges gazeux de la charge et les résistances, sont, de préférence, de forme tubulaire. Les gaines sont constituées par un matériau réfractaire ; on peut utiliser des céramiques comme la mullite, la cordiérite, le nitrure de silicium, le carbure de silicium, la silice ou l'alumine ; le carbure de silicium est le matériau préférentiellement choisi car il présente une bonne conductivité thermique.

La distance qui sépare les éléments chauffants des gaines de céramique est fonction de la section de l'élément chaufant. Pour des résistances de forme cylindrique de diamètre d, on utilise des gaines tubulaires de diamètre D généralement compris entre 1,2 d et 4 d, et de préférence, compris entre 1,5 d et 3 d.

L'espace compris entre un élément de chauffage et la gaine céramique qui l'entoure est préférentiellement rempli par un fluide gazeux tel que l'azote, le gaz carbonique ou l'air pour mieux conduire la chaleur et augmenter la durée de vie de l'élément de chauffage.

Les éléments de chauffage sont disposés en nappes parallèles sensiblement perpendiculaires au sens d'écoulement de la charge, préférentiellement en quinconce, de façon à ce que la distance qui sépare deux gaines voisines soit la plus réduite possible, tout en tenant compte des impératifs de perte de charge admissible ; elle sera généralement comprise entre 1 et 100 mm, et de préférence entre 2 et 20 mm.

Selon un mode de réalisation de l'invention, les espaces libres définis par les nappes de gaines céramiques, destinés à la circulation de la charge, sont au moins partiellement occupés par des garnissages, généralement en céramique préférentiellement conducteurs de la chaleur.

On peut ainsi, pour un type de réacteur donné, diminuer le temps de séjour d'une charge dans ce réacteur, tout en homogénéisant l'écoulement du mélange gazeux et en répartissant mieux la chaleur dissipée.

Ils peuvent avoir des formes diverses, et se présenter par exemple sous forme d'anneaux de raschig, de selles de Berl, d'anneaux de Lessing ou d'anneaux de Pall, de barreaux, de tubes cylindriques fermés.

L'invention sera mieux comprise au vu des figures représentant à titre illustratif et non limitatif des modes de réalisation ou de l'invention :

– La Figure 1A représente une coupe longitudinale du réacteur suivant un plan perpendiculaire de l'axe de symétrie des gaines en céramique, et la figure 1C une coupe longitudinale suivant l'axe de symétrie des gaines.

– La Figure 1B représente une coupe longitudinale du réacteur avec garnissage.

– La Figure 2 illustre un détail de réalisation de la zone de chauffage dans un plan identique à la Figure 1C.

Sur la figure 1A, on a représenté, selon un mode de réalisation, un réacteur 1 vertical de forme allongée et de section rectangulaire, comprenant un distributeur 2 permettant d'alimenter par un orifice d'entrée 5 le réacteur en mélange gazeux réactionnel. Ce dernier, qui contient par exemple 50 % de méthane, a été préchauffé dans une zone de préchauffage conventionnelle, non représentée sur la figure, de préférence par convection. Le réacteur comprend une pluralité de moyens de chauffage électriques 3 entourés de gaines céramique 4, disposés en nappes parallèles et formant dans un plan (plan de la figure) un faisceau à pas triangulaire. Ces nappes définissent des sections de chauffage transversales sensiblement perpendiculaires à l'axe du réacteur défini selon la direction d'écoulement de la charge.

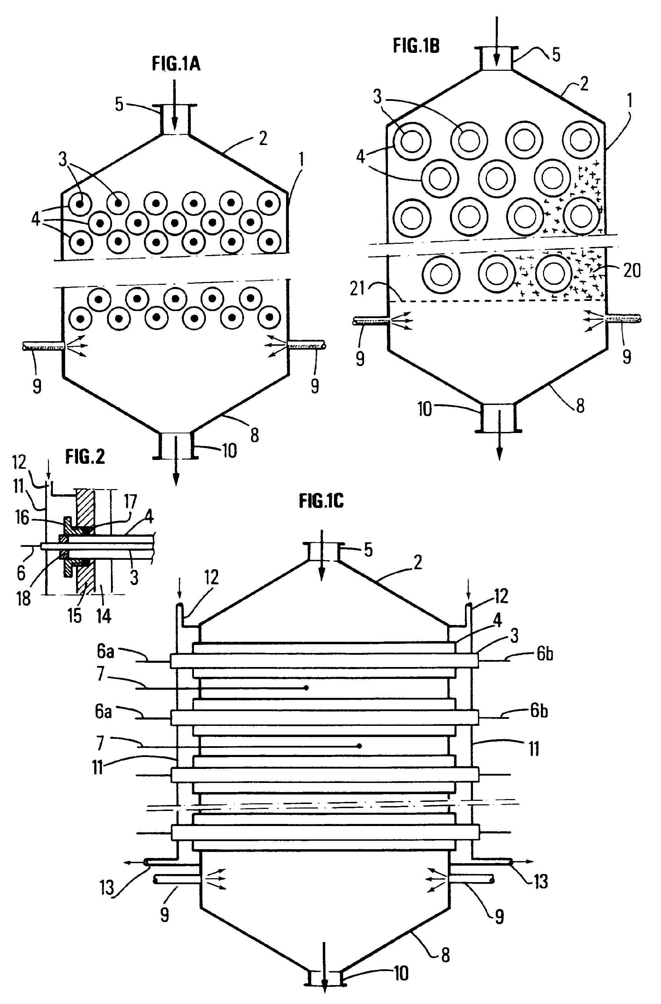

Ces sections de chauffage sont alimentées en énergie électrique, de façon indépendante, grâce à une paire d'électrodes (6a, 6b sur la Figure 1C).

Des sondes pyrométriques à thermocouple (7, Figure 1C) sont logées dans les espaces où circule la charge entre les gaines 4 et permettent de réguler automatiquement la température de chaque section de chauffage, par un dispositif classique de régulateur et de modulateur non représenté sur la figure.

Dans la première partie de la zone de chauffage, les gaines en céramique sont chauffées de façon à ce que la température de la charge passe rapidement de 750 °C (température de préchauffage) à 1 200 °C environ ; cette zone de chauffage progressif représente en général environ 65 % de la longueur de la zone de chauffage ; le mélange gazeux circule ensuite dans la deuxième partie de la zone de chauffage, où l'on maintient généralement la température à une valeur constante sensiblement égale à celle atteinte à la fin de la première zone de chauffage, soit en général 1 200 °C environ. A cet effet, on module la puissance électrique fournie à plusieurs sections de chauffage qui constituent la deuxième partie de la zone de chauffage ; on parvient ainsi à obtenir une variation de température inférieure d'environ 10 °C autour de la valeur de consigne. La longueur de cette deuxième zone de chauffage représente environ 35 % de la longueur totale de la zone de chauffage.

Selon la figure 1B, ce réacteur peut comporter des moyens de chauffage électriques 3 entourés de gaines 4 en matière céramique. L'espace entre les gaines est rempli d'un garnissage avantageusement en céramique qui est retenu par une grille 21 à l'extrémité de la zone de chauffage.

A la sortie de la zone de chauffage, les effluents de la réaction sont refroidis dans une zone de refroidissement 8. Ils sont mis en contact avec un agent de trempe tel que du propane introduit par l'intermédiaire d'injecteurs 9 de trempe disposés à la périphérie du réacteur 1 et reliés à une source extérieure de propane non représentée. L'ensemble des gaz effluents est refroidi à une température d'environ 500 °C et recueilli par un orifice de sortie 10 à l'extrémité de la zone réactionnelle 1.

Selon un autre mode de réalisation non illustré, les effluents peuvent être refroidis en circulant à travers des conduits étanches disposés dans la zone 8 par lesquels s'écoule l'agent de trempe, ces conduits étant reliés à la source extérieure de l'agent de trempe.

- La Figure 1C représente les mêmes éléments que ceux décrits dans la Figure 1A ; on a représenté, de plus, les boitiers de protection 11, munis d'orifice 12 et 13 permettant la circulation dans les boitiers 11 d'un gaz inerte tel qu'azote ou gaz carbonique par exemple. Ces boitiers sont fixés sur l'armature métallique du réacteur et entourent l'ensemble des résistances électriques et des gaines céramiques, à l'exception de l'extrémité des résistances électriques, par où se fait l'alimentation. La circulation de gaz inerte est habituellement effectuée en légère surpression par rapport au réacteur, assurant ainsi une atmosphère parfaitement contrôlée.

La Figure 2 représente un détail du mode de réalisation de la zone de chauffage suivant l'invention. On utilise comme moyen de chauffage électrique des résistances en carbure de silicium, de forme cylindriques 3. Ces résistances comportent à chacune de leur extrémité des zones froides, et une partie centrale qui est la zone chaude représentant par exemple environ 68 % de la longueur totale.

On réalise un réacteur de section rectangulaire, dont les parois sont constituées par un béton réfractaire isolant 14, et par une armature métallique 15. On perce dans deux parois latérales opposées un trou circulaire, dans lequel on fait passer une gaine en céramique 4, de diamètre double de celui de la résistance électrique 3. L'étanchéité de la gaine céramique est assurée au niveau de l'armature métallique 15 qui est munie d'une gorge permettant le serrage d'une tresse en céramique 17 au moyen d'un système presse-étoupe 16. Le positionnement de la résistance 3 dans la gaine 4 est effectué au moyen de rondelles en fibre céramique 18.

La zone chaude de la résistance 3 est positionnée de façon à ce qu'elle ne pénètre pas dans l'orifice de passage à travers la paroi de béton isolant.

On dispose ainsi un certain nombre de résistances chauffantes gainées dans des manchons en céramique, par rangées horizontales successives, ces rangées étant décalées de façon à ce que, sur les parois latérales du four, elles forment un faisceau à pas triangulaire. Un boitier de protection 11 dont dépassent seulement les extrémités des résistances et leur alimentation électrique 6, est parcouru par un courant de gaz inerte 12.

EXEMPLE

On utilise un réacteur à trempe indirecte, de longueur totale de 5,80 m et de section rectangulaire de 2 x 3,2 m. Les moyens de chauffage de ce réacteur sont constitués par des résistances électriques en céramique, de marque KANTHAL ; ces résistances sont entourées de gaines en céramique, disposées concentriquement par rapport à ces résistances.

Les gaines sont disposées en nappes parallèles, disposées perpendiculairement au sens de circulation de la charge, et forment en projection perpendiculaire un faisceau à pas carré (disposition en quinconce). La longueur d'une résistance électrique est de 2 m, et son diamètre est de 54 mm. Les gaines en céramique ont une longueur de 2 m et un diamètre extérieur de 150 mm ; la distance séparant deux gaines voisines est 10 mm.

La première partie de la zone de chauffage, longue de 3,70 m, comprend 23 nappes de résistances, chaque nappe comprenant 20 résistances ; dans cette zone, la charge, préchauffée à 800 °C, est portée à 1 200 °C. Cette zone est régulée thermiquement par l'intermédiaire de thermocouples disposés dans les espaces où circule la charge.

La deuxième partie de la zone de chauffage, qui est adjacente à la zone réactionnelle, est longue de 1,75 m ; elle est constituée de 15 nappes de 20 résistances, disposées de la même façon que dans la zone de chauffage. Cette zone est constituée par 5 sections de chauffage, régulées indépendamment, permettant d'assurer le maintien de la température dans cette zone à 1 200 plus ou moins 10 °C.

Les gaz effluents sont refroidis dans un premier temps à 800 °C par échange indirect avec les gaz de charge ; d'autres échangeurs de température permettent ensuite d'abaisser leur température à 300 °C environ.

On utilise comme charge du méthane dilué avec de l'hydrogène dans un rapport équimoléculaire. Ce mélange est préchauffé à 800 °C et craqué dans le réacteur décrit ci-dessus, avec un temps de séjour dans la zone réactionnelle de 50 ms.

Après refroidissement à température ambiante, pour 200 moles de mélange équimoléculaire de méthane et d'hydrogène, on obtient les produits suivants :

| PRODUITS | QUANTITES |
|----------|-----------|
| $H_2$ | 163,45 moles |
| $CH_4$ | 55 moles |
| $C_2H_2$ | 5,9 moles |
| $C_2H_4$ | 7,2 moles |
| Benzène | 2,15 moles |
| Coke | 72 g |

**Revendications**

1. Procédé de conversion thermique de méthane en hydrocarbures de poids moléculaire plus élevé dans une zone de réaction allongée ayant un axe de symétrie et comprenant une zone de chauffage et une zone de refroidissement, faisant suite à ladite zone de chauffage et dans laquelle on refroidit les effluents de la zone de chauffage, caractérisé en ce que l'on fait circuler un mélange gazeux renfermant du méthane dans ladite zone de chauffage selon une direction d'écoulement sensiblement parallèle à l'axe de ladite zone de réaction, la zone de chauffage contenant une pluralité de moyens de chauffage électriques étanches vis à vis du mélange gazeux, disposés en nappes parallèles formant en projection transversale un faisceau à pas triangulaire, carré ou rectangulaire, lesdits moyens de chauffage étant regroupés par sections successives transversales sensiblement perpendiculaire à l'axe, indépendantes entre elles et alimentées en énergie électrique de façon à déterminer deux parties dans la zone de chauffage, la première partie de la zone permettant de porter la charge jusqu'à une température au plus égale à 1 500 °C, la deuxième partie, subséquente de la première, maintenant la charge à une température sensiblement égale à la température maximum à laquelle elle a été portée dans la première partie de façon à ce que la variation de température tout au long de ladite deuxième partie de la zone de chauffage soit inférieure à environ 50°C et de préférence 10°C, le procédé étant aussi caractérisé en ce qu'on introduit un fluide de refroidissement dans ladite zone de refroidissement et en ce qu'on recueille lesdits hydrocarbures de poids moléculaires plus élevés à l'extrémité de la zone réactionnelle.

2. Procédé selon la revendication 1 dans lequel on chauffe une première partie de la zone de chauffage jusqu'à une température maximale au plus égale à 1 500 °C de façon à réaliser un gradient thermique compris entre 0,5 °C/cm et 25 °C/cm sur une longueur comprise entre 20 et 80 % de la zone totale de chauffage et dans lequel on chauffe une deuxième partie subséquente de la dite première partie de façon à ce que la variation de température tout au long de ladite deuxième partie de la zone de chauffage soit inférieure à environ 50° C et de préférence 10° C.

3. Procédé selon la revendication 1 ou 2 dans lequel on met en contact indirect lesdits effluents de la zone de chauffage avec ledit fluide de refroidissement et on recueille lesdits hydrocarbures de poids moléculaire plus élevés.

4. Procédé selon l'une quelconque des revendications 1 à 3 dans lequel lesdits moyens de chauffage sont cylindriques et dans lequel on isole lesdits moyens de chauffage par des gaines cylindriques concentriques, en matière céramique, le rapport du diamètre des gaines au diamètre des moyens de chauffage étant compris entre environ 1,2 et 4 et de préférence entre environ 1,5 et 3.

5. Procédé selon la revendication 4 dans lequel on fait circuler ledit mélange gazeux dans des passages entre lesdites gaines dont la distance est comprise entre 1 et 100 mm.

6. Procédé selon la revendication 5 dans lequel lesdits passages sont sensiblement identiques.

7. Dispositif pour la mise en oeuvre du procédé selon l'une des revendications 1 à 6 comprenant des moyens d'alimentation en mélange gazeux, des moyens d'évacuation des effluents produits et des moyens d'alimentation d'un fluide de refroidissement, caractérisé en ce qu'il comprend un réacteur (1) de forme allongée, de préférence à section carrée ou rectangulaire, en matière réfractaire, ayant un axe de symétrie et relié d'une part, à une première extrémité, auxdits moyens d'alimentation en mélange gazeux et d'autre part, à l'extrémité opposée, auxdits moyens d'évacuation, le dit réacteur comprenant sur une première partie (coté première extrémité) une pluralité de moyens de chauffage électriques (3) entourés de façon étanche de gaines en céramique (4), lesdits moyens sensiblement parallèles entre eux étant disposés en nappes sensiblement parallèles, perpendiculaires à l'axe de symétrie du réacteur de façon à définir entre les gaines des espaces ou passages pour la circulation des mélanges gazeux et/ou des effluents, lesdits moyens de chauffage et lesdites gaines étant adaptés à chauffer lesdits passages par sections transversales successives indépendantes sensiblement perpendiculaires à l'axe du réacteur, ledit réacteur comportant en outre des moyens d'asservissement et de modulation de chauffage reliés auxdits moyens de chauffage, ledit réacteur comportant par ailleurs sur une deuxième partie (8) (côté extrémité opposée) contigüe à la première partie des moyens de refroidissement (9) des effluents reliés auxdits moyens d'alimentation du fluide de refroidissement.

8. Dispositif selon la revendication 7 dans lequel les moyens de chauffage électrique et les gaines en céramique qui les entourent sont cylindriques et concentriques, le rapport du diamètre des gaines au diamètre des moyens de chauffage étant compris entre 1,2 et 4 et de préférence entre 1,5 et 3.

9. Dispositif selon l'une des revendications 7 à 8 dans lequel la distance séparant deux gaines est comprise entre environ 1 et 100 mm et de préférence entre 2 et 20 mm.

10. Dispositif selon l'une des revendications 7 à 9 dans lequel lesdits passages sont au moins partiellement remplis de garnissage en céramique.


**Patentansprüche**

1. Verfahren zur thermischen Umwandlung von Methan in Kohlenwasserstoffe mit höherem Molekulargewicht in einer länglichen Reaktionszone mit einer Symmetrieachse und mit einer Heizzone sowie einer an diese Heizzone sich anschliessenden Kühlzone in welcher man die Abströme aus der Heizzone kühlt, dadurch gekennzeichnet, daß man ein gasförmiges Methan enthaltendes Gemisch in dieser Heizzone in einer Strömungsrichtung im wesentlichen parallel zur Achse dieser Reaktionszone zirkulieren läßt, wobei die Heizzone eine Vielzahl von elektrischen gegenüber dem gasförmigen Gemisch dichten Heizmitteln enthält, die in parallelen Bahnen angeordnet sind und in der Transversalprojektion ein Bündel mit dreieckiger, quadratischer oder rechtwinkeliger Teilung bilden, wobei diese Heizmittel zu aufeinanderfolgenden transversalen Abschnitten im wesentlichen senkrecht zur Achse zusammengefaßt und unabhängig untereinander sind und mit elektrischer Energie derart versorgt werden, daß zwei Teile in der Heizzone bestimmt werden, wobei der erste Teil der Zone es ermöglicht, die Charge bis auf eine Temperatur von höchtens 1500°C zu bringen und der zweite auf den ersten folgende Teil die Charge bei einer Temperatur im wesentlichen gleich der Maximaltemperatur hält, auf die sie in dem ersten Teil gebracht worden ist, derart, daß die Veränderung der Temperatur längs des gesamten zweiten Teils der Heizzone kleiner als etwa 50°C und vorzugsweise 10°C ist, wobei das Verfahren sich auch dadurch auszeichnet, daß man ein Kühlfluid in diese Kühlzone einführt und daß man diese Kohlenwasserstoffe mit höheren Molekulargewichten am Ende der Reaktionszone sammelt.

2. Verfahren nach Anspruch 1, bei dem man einen ersten Teil der Heizzone bis auf eine Maximaltemperatur von höchstens 1500°C derart erwärmt, daß ein Wärmegradient zwischen 0,5°C/cm und 25°C/cm auf eine Länge realisiert wird, die zwischen 20 und 80% der gesamten Heizzone umfaßt und bei dem man einen zweiten auf den ersten Teil folgenden Teil derart erhitzt, daß die Veränderung der Temperatur längs des gesamten zweiten Teils der Heizzone kleiner als etwa 50°C und vorzugsweise 10°C ist.

3. Verfahren nach Anspruch 1 oder 2, bei dem man diese Abströme aus der Heizzone in indirekten Kontakt mit diesem Kühlfluid setzt und man diese Kohlenwasserstoffe mit erhöhten Molekulargewichten sammelt.

4. Verfahren nach einem der Ansprüche 1 bis 3, bei dem diese Heizeinrichtungen zylindrisch sind und bei dem man diese Heizmittel durch zylindrische konzentrische Mäntel aus keramischem Material isoliert, wobei das Verhältnis des Durchmessers der Mäntel zum Durchmesser der Heizmittel zwischen etwa 1,2 und 4 und vorzugsweise zwischen etwa 1,5 und 3 beträgt.

5. Verfahren nach Anspruch 4, bei dem man dieses gasförmige Gemisch in den Durchlässen zwischen diesen Mänteln, deren Entfernung zwischen 1 und 100 mm beträgt, zirkulieren läßt.

6. Verfahren nach Anspruch 5, bei dem diese Durchlässe im wesentlichen identisch sind.

7. Vorrichtung zur Durchführung des Verfahrens nach einem der Ansprüche 1 bis 6, mit Speisemitteln für das gasförmige Gemisch, Mitteln zum Abziehen der erzeugten Abströme und Mitteln zum Speisen mit einem Kühlfluid, dadurch gekennzeichnet, daß sie einen Reaktor (1) länglicher Form, vorzugsweise mit quadratischem oder rechteckigem Querschnitt aus feuerfestem Material umfaßt, der eine Symmetrieachse hat und einerseits an einem ersten Ende mit diesen Speisemitteln für das gasförmige Gemisch und andererseits am gegenüberliegenden Ende mit diesen Abzugsmitteln verbunden ist, wobei dieser Reaktor auf einem ersten Teil (Seite des ersten Endes) eine Vielzahl von elektrischen Heizmitteln (3) umfaßt, die dicht von Mänteln aus Keramik (4) umgeben sind, wobei diese untereinander im wesentlichen parallele Mittel in im wesentlichen parallelen Bahnen senkrecht zur Symmetrieachse des Reaktors derart angeordnet sind, daß zwischen den Mänteln Räume oder Durchlässe für die Zirkulation der gasförmigen Gemische und/oder Abströme definiert werden, wobei diese Heizmittel und diese Mäntel so ausgelegt sind, daß sie diese Durchlässe durch aufeinanderfolgende unabhängige transversale Abschnitte im wesentlichen senkrecht zur Achse des Reaktors erwärmen, wobei der Reaktor im übrigen Hilfssteuer- und Heizmodulationsmittel umfaßt, die mit diesen Heizmitteln verbunden sind, wobei der Reaktor im übrigen auf einem zweiten Teil (8) (Seite des gegenüberliegenden Endes) angrenzend an den ersten Teil Kühlmittel (9) der Abströme umfaßt, die mit diesen Speisemitteln für das Kühlfluid verbunden sind.

8. Vorrichtung nach Anspruch 7, bei dem die Mittel zur elektrischen Heizung und die Mäntel aus Keramik, die sie umschließen, zylindrisch und konzentrisch sind, wobei das Verhältnis des Durchmessers der Mäntel zum Durchmesser der Heizmittel zwischen 1,2 und 4 und vorzugsweise zwischen 1,5 und 3 beträgt.

9. Vorrichtung nach einem der Ansprüche 7 bis 8, bei dem die zwei Mäntel trennende Entfernung zwischen etwa 1 und 100 mm und vorzugsweise zwischen 2 und 20 mm beträgt.

10. Vorrichtung nach einem der Ansprüche 7 bis 9, bei der diese Durchlässe wenigstens teilweise zur Auskleidung aus Keramik gefüllt sind.


## Claims

1. Process for the thermal conversion of methane into higher molecular weight hydrocarbons in an elongated reaction zone having an axis of symmetry and incorporating a heating zone and a cooling zone following onto said heating zone and in which the effluents of the heating zone are cooled, characterized in that a methane-containing gaseous mixture is made to flow in said heating zone in accordance with a flow direction which is substantially parallel to the axis of said direction zone, the heating zone containing a plurality of electrical heating means which are tight with respect to the gaseous mixture and arranged in parallel layers forming in transverse projection a bundle having a triangular, square or rectangular spacing, said heating means being grouped by successive, transverse sections substantially perpendicular to said axis of symmetry, which are independent of one another and supplied with electrical power in such a way as to define two portions in the heating zone, the first portion of the zone making it possible to raise the charge to a temperature at the most equal to 1500°C and the second portion, following onto the first portion, maintaining the charge at a temperature substantially equal to maximum temperature to which it has been raised in the first portion, so that the temperature all along said second portion of the heating zone is below approximately 50°C and preferably 10°C, the process also being characterized in that a cooling fluid is introduced into said cooling zone and that said higher molecular weight hydrocarbons are collected at the end of the reaction zone.

2. Process according to claim 1, wherein a first portion of the heating zone is heated to a maximum temperature of at the most 1500°C, so as to obtain a thermal gradient between 0.5 and 25°C/cm over a length between 20 and 80% of the total heating zone and wherein a second portion following the first portion is heated in such a way that the temperature variation along said second portion of the heating zone is below approximately 50°C and preferably 10°C.

3. Process according to claim 1 or 2, wherein the effluents of the heating zone are indirectly contacted with the cooling fluid and the higher molecular weight hydrocarbons are collected.

4. Process according to any one of claims 1 to 3, wherein the heating means are cylindrical and wherein the said heating means are insulated by ceramic material, concentric, cylindrical sheaths, the ratio of the diameter of the sheaths to the diameter of the heating means being between approximately 1.2 and 4 and preferably between approximately 1.5 and 3.

5. Process according to claim 4, wherein the gaseous mixture is circulated in the passages between the said sheaths, whose spacing is between 1 and 100 mm.

6. Process according to claim 5, wherein the said passages are substantially identical.

7. Apparatus for performing the process according to any one of the claims 1 to 6 comprising means for supplying the gaseous mixture, means for discharging the effluents produced and means for supplying a cooling fluid, characterized in that it comprises an elongated reactor (1), having a preferably square or rectangular cross-section, made from refractory material, having an axis of symmetry and connected on the one hand, at a first end, to the said gaseous mixture supply means and on the other hand, at the opposite end, to the said discharge means, the reactor having on a first portion (first end side) a plurality of electrical heating means (3) tightly surrounded by ceramic sheaths (4), said substantially parallel means being arranged in the form of substantially parallel layers perpendicular to the axis of symmetry of the reactor, so as to define between the sheaths spaces or passages for the circulation of gaseous mixtures and/or effluents, said heating means and said sheaths being able to heat the said passages by successive independent cross-sections substantially perpendicular to the reactor axis, the reactor also having heating control and modulating means connected to said heating means, the reactor also having on a second portion (8) (opposite end side) contiguous to the first portion means (9) for cooling the effluents connected to said cooling fluid supply means.

8. Apparatus according to claim 7, wherein the electrical heating means and the ceramic sheaths surrounding them are cylindrical and concentric, the ratio of the diameter of the sheaths to the diameter of the heating means being between 1.2 and 4 and preferably between 1.5 and 3.

9. Apparatus according to one of the claims 7 and 8, wherein the distance separating two sheaths is between approximately 1 and 100 mm and preferably 2 and 20 mm.

10. Apparatus according to any one of the claims 7 to 9, wherein the said passages are at least partly filled with a ceramic lining.

FIG.1A

FIG.1B

FIG.2

FIG.1C